(19) **Europäisches Patentamt**

**European Patent Office**

**Office européen des brevets**

(11) **EP 1 122 678 A2**

(12) # EUROPEAN PATENT APPLICATION

(43) Date of publication:
**08.08.2001   Bulletin 2001/32**

(51) Int Cl.7: **G06F 19/00**

(21) Application number: **01300829.7**

(22) Date of filing: **31.01.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.01.2000   US 494476**

(71) Applicant: **Panmedix Inc.**
**New York, NY 10038 (US)**

(72) Inventors:
• **Comrie, McDonald**
  **Staten Island, NY 10301 (US)**

• **Erlanger, David Michael**
  **New York, NY 10024 (US)**
• **Kaplan, Darin F.**
  **New York, NY 10025 (US)**
• **Theodoracopulos, Alexis**
  **New York, NY 10021 (US)**
• **Yee, Philip**
  **New York, NY 10013 (US)**
• **Shchogolev, Vladislav**
  **Brooklyn, New York 11229 (US)**

(74) Representative: **W.P. Thompson & Co.**
**Coopers Building,**
**Church Street**
**Liverpool L1 3AB (GB)**

(54)   **Measuring pharmaceutical cognitive impairment**

(57)   Inventions to quantify cognitive effects associated with drugs.

*Figure 1*

EP 1 122 678 A2

**Description**

BACKGROUND

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

Cognitive dysfunction in its various forms poses a significant public health problem. Drugs avail to treat certain forms of cognitive dysfunction. While such pharmaceutical products are useful, they often have adverse side effects which become more severe as larger doses of the drug are used by the patient. Thus, to get the optimum therapeutic response from a specific patient, the drug needs to be administered in a dosage large enough to obtain efficacy against cognitive dysfunction, but small enough to minimize the severity of the drug's unwanted or adverse side effects.

For a particular drug product, the optimal dosage amount varies from patient to patient. The amount varies depending on a number of factors, including the patient's weight, age, genetic makeup, stage of the disease, etc.... To administer such drugs, the practice standard in the art has been to prescribe an initial drug dosage based on the patient's weight or age.

Such initial drug dosage may be subsequently adjusted, or "titrated." Drug dosages optimally should be carefully, and perhaps repeatedly, adjusted for each patient, over the course of time, to assure that the patient receives a drug dosage large enough to give the most therapeutic benefit, without incurring an unwarranted severity of adverse side effects. Titration is now done based on the results of biological (e.g., hair follicle or blood serum) sample testing or a physician's somewhat subjective evaluation of the patient, including the patient's responses to personally-administered cognitive tests. These methods of titration are expensive, potentially painful for the patient, and not completely accurate.

For example, the symptoms of attention deficit disorder are treatable with a stimulant, $\alpha$-phenyl-2-piperidine-acetic acid methyl ester (commonly called "methylphenidate," commercially avaiable under the trademark RITALIN (TM), from Novartis Pharmaceutical Corporation, East Hanover, New Jersey). The use of methylphenidate to treat the symptoms of attention deficit disorder is described in Pelham, W.E. et al., Journal of Consulting Clinical Psychology, v. 61, pp. 506 et *seq.* (1993); Klein, R.G., Encephale, v. 19, pp. 89 et *seq.* (1993).

If the patient is given too little methylphenidate, however, then the patient may not have sufficient therapeutic benefit, and may thus consequently exhibit the low attention levels symptomatic of attention deficit disorder. If, however, the same patient is given too much methylphenidate, then the patient may have excessively severe side effects such as reduction in physical growth, depression or sadness, headache, anxiety or hyperactivity. With methylphenidate, this dosage adjustment is now done by subjecting the patient to biological sampling, to assess the physical concentration of certain methylphenidate metabolic products in the patient.

The same need to determine optimal drug dosing exists to some extent for each individual patient and for any drug, including many pharmaceuticals available to treat cognitive dysfunction. For example, another drug used to treat the symptoms of attention deficit disorder is an amphetimine commonly called "adderall," which must be administered in optimal dosages to achieve optimal effects.

Determining for an individual patient the correct dosage of a drug, can be problematic because it can be difficult to assess the existence and severity of the drug's effect on the patient's cognition *quantitatively.* The assessment now typically involves a physician manually showing a patient a series of picture cards or words, and tallying the patient's ability to correctly respond to these images. Such assessment is done by hand, administered individually and personally to a patient by a psychiatrist or health worker. Such personally administered testing is time intensive and therefore somewhat expensive. Further, the results of such assays vary depending on the identity and psychiatric experience of the specific physician or health care worker administering the assay. Thus, results obtained by different physicians, often working in different locations, are often difficult to compare. There has thus been a need for a way to measure the cognitive effect of drugs directly, by their effect on cognitive dysfunction, rather than indirectly, as by the physical presence of drug residues or metabolism byproducts.

Published patents include the following:

Steven J. Brown discloses a method for diagnosis and treatment of psychological and emotional disorders using a microprocessor based video game, United States Letters Patent No. 5,913,310. Disclosed examples include "schizophrenia, depression, hyperactivity, phobias, panic attacks, anxiety, overeating, and other psychological disorders" such as "personality disorders, obsessive-compulsive disorders, hysteria, and paranoia."

Joel S. Douglas et al. discloses an analyte testing system with test strips, United States Letters Patent No. 5,872,713. These test strips are effective to assay for certain chemical changes in a patient, but have not been used to assay cognitive dysfunction or impairment.

Patrick Lichter discloses a personal computer card for collecting biological data, United States Letters Patent No. 5,827,179. The patent claims a portable computer card used with an air pressure transducer, see id. at claim 1, or a "biological data receiver," see id. at claim 6. The biological data receiver "can be adapted to receive biological data from a pulse oximetry sensor" or "from an ECG sensor." This system may be effective to assay for certain physical changes in a patient such as pulmonary function, but it has not been used to assay cognitive dysfunction or impairment.

Tom Marlin discloses a system for constructing formulae for processing medical data, United States Letters Patent No. 5,715,451. The patent says that rather than providing a prepared statistical analysis package, Marlin discloses a computer interface to construct statistical and other mathematicla formulae to ease the analysis of clinical data.

Stephen Raymond et al. discloses a health monitoring system that "tracks the state of health of a patient and compiles a chronological health history ... us[ing] a multiparametric monitor which ... automatically measures and records a plurality of physiological data from sensors in contact with the patient's body," wherein "[t]he data collected is not specifically related to a particular medical condition" such as cognitive dysfunction. United States Letters Patent No. 5,778,882.

William Reber et al. discloses a medical communication timing system comprising an output device that generates an alert for the proper time to take a first medicine and a second medicine. United States Letters Patent No. ; 5,950,632 and United States Letters Patent No. 5,950,632. Michael Swenson et al. discloses a virtual medical instrument for performing medical diagnostic testing, United States Letters Patent No. 5,623,925 and United States Letters Patent No. 5,776,057. The instrument "includes a universal interface having a number of electrical contacts and sets of electrical conduits associated with the different stored diagnostic test protocols. * * * The system is constructed to enable the selected diagnostic test protocol to be performed on a patient after the corresponding set of electrical conduits are connected to the universal interface contacts and to the patient."

Paul Tamburini et al. discloses a diagnostic assay for Alzheimer's disease based on the proteolysis of the amyloid precursor protein, United States Letters Patent No. 5,981,208. This assay may be effective to assay for certain physical changes in a patient, but it has not been used to directly measure cognitive dysfunction or impairment, and thus has not been shown effective to titrate the administration of drugs to treat cognitive dysfunction.

Not disclosed in the prior art - nor even suggested in it - is our non-invasive, computerized invention to accurately measure the cognitive impact of pharmaceuticals, and to thereby adjust the dosage of the pharmaceutical. There is a need for such an invention that is easy to administer, rapid, less expensive, and accurate enough to enable adjusting the amount of such pharmaceuticals to the most beneficial dosage amount and administration schedule.

SUMMARY

We have invented a solution that enables one to -better assess the impact of drugs for cognitive dysfunction. Our solution works at minimal cost per administration, compared to a roughly 55% to 65% accuracy and approximately $2,000 cost per assay for the personally-administered assays previously known in the art.

Further, our assay shows consistent results regardless of the identity or experience level of the treating physician. Thus, the results obtained from our test can be pooled and compared among patients located in different study locations, with a level of data comparability and accuracy not before available in the art. This data comparability greatly improves the usefulness and informativeness of the test responses. It improves the accuracy of the diagnosis of cognitive impairment in an individual patient. It enables drug clinical research done at different locations, and by different health care practitioners, to be easily compared. It improves the ability of pharmaceutical research scientists to correctly evaluate the cognitive impact of pharmaceuticals.

BRIEF DESCRIPTION OF THE FIGURES

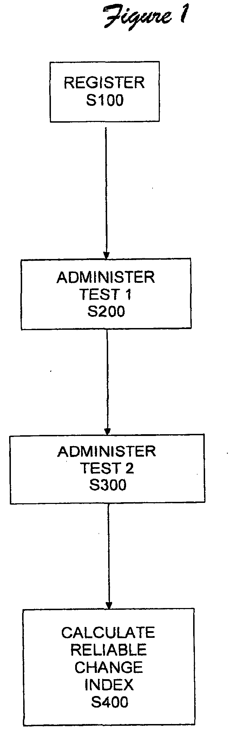

FIGURE 1 shows an overview of the process, divided into four parts.

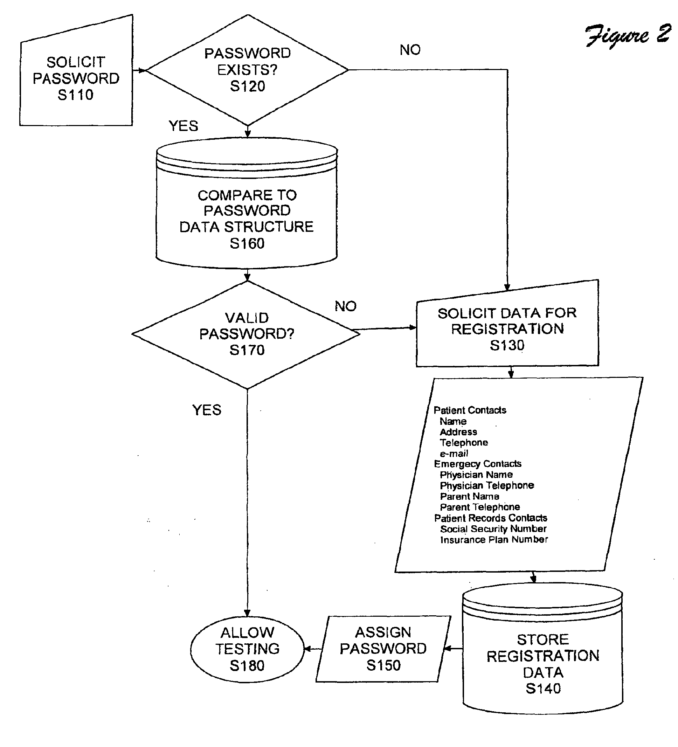

FIGURE 2 illustrates the first part of the process.

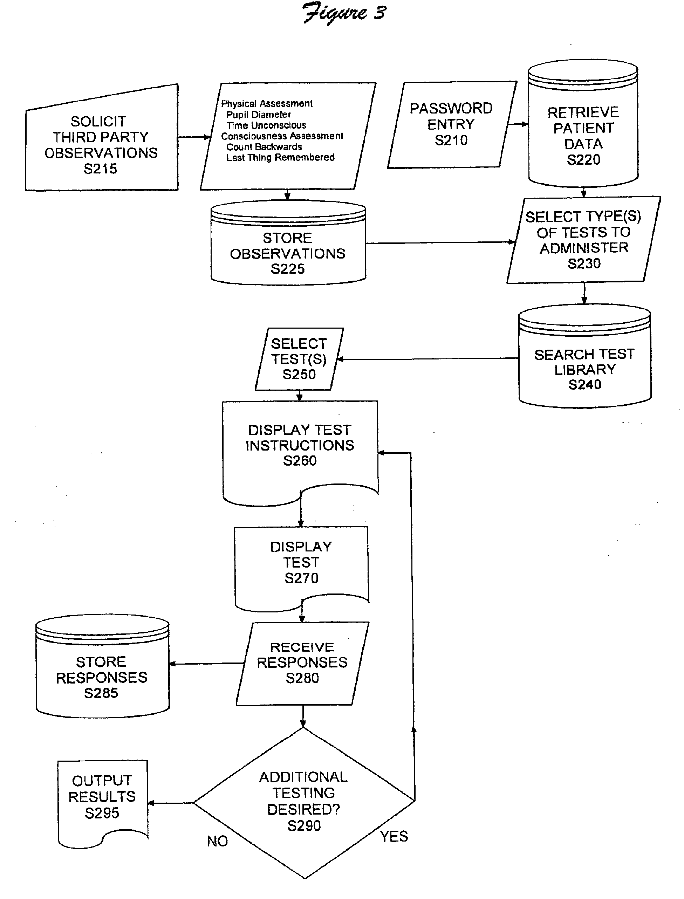

FIGURE 3 illustrates the second part of the process.

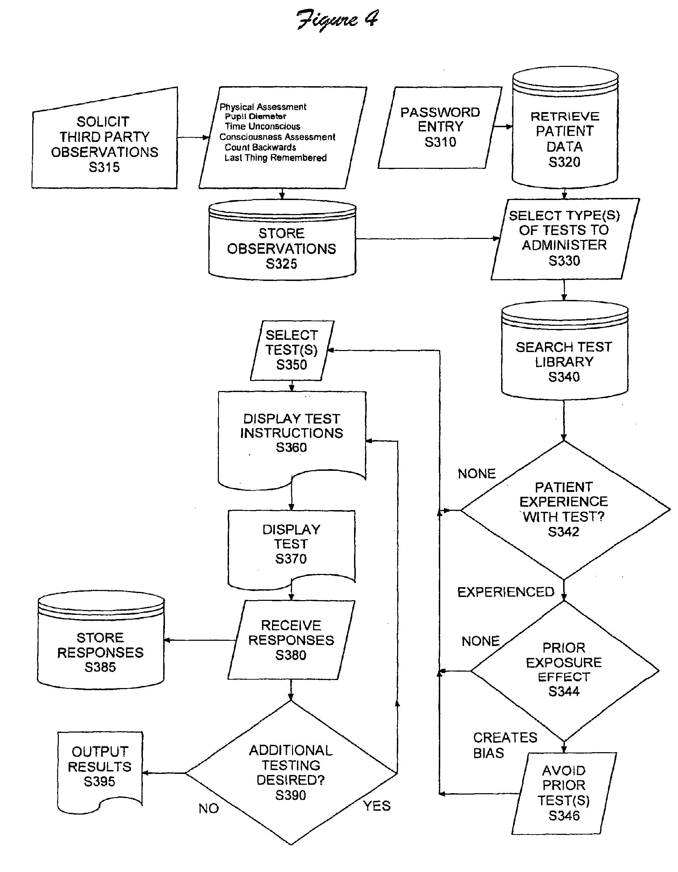

FIGURE 4 illustrates the third part of the process.

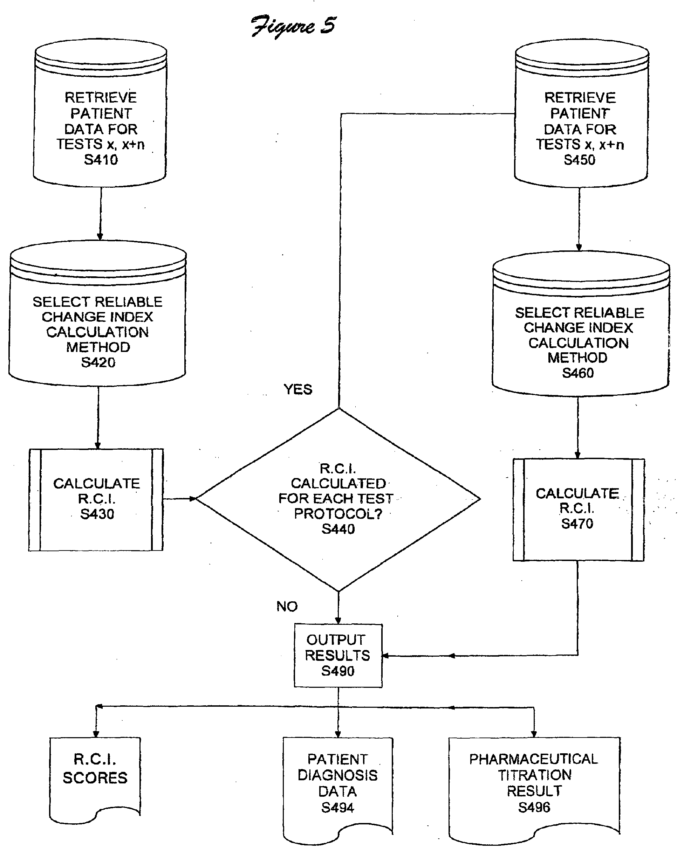

FIGURE 5 illustrates the fourth part of the process.

DETAILED DESCRIPTION

Our invention generally entails using a computer to show a patient taking a pharmaceutical product a series of cognitive dysfunction tests, receiving the patient's test responses, and analyzing these responses to assess cognitive dysfunction in the patient, whereby a conclusion regarding whether symptoms of cognitive dysfunction probably exist or are absent in the patient, and the drug's likely causal effect on cognitive dysfunction. Saliently, in contrast to the prior art, which teaches manual, one-time testing, our invention enables the comparison of multiple test results over time, to assess the change over time in a patient's responses. The patient's degree and rate of change over time is, in certain ways, significantly more informative that a static, one-time score.

The term "cognitive dysfunction testing protocol" is used herein to connote cognitive testing protocols to measure cognitive functions (immediate and short-term memory and pattern recognition, for example) by providing the patient or user with a series of sensory stimuli, and measuring the user's ability to consciously and voluntarily respond to and remember said stimuli. To make our invention, one can use any of a wide variety of cognitive function testing protocols. Examples known in the art include the series of handheld flash cards developed by Professor Jeffrey T.

Barth of The University of Virginia, and similar flash cards commercially available from The Psychological Corporation, a division of Harcourt Brace Jovanovich Publishers, New York, New York. The precise identity of the testing protocols is not important.

In our preferred embodiment, the cognitive dysfunction testing protocols are visual or auditory. That is to say, they entail showing the user a series of images or sounds and measuring the user's ability to remember and respond to these. We disclose and discuss below the specific details of some examples of cognitive dysfunction testing protocol. Our invention is not, however, limited, to these specific testing protocols disclosed below. One can readily make. our invention using other visual testing protocols. One can even make versions of our invention using other types of sensory response protocols. For example, one can make our invention using auditory stimuli, in place of visual stimuli. This may be necessary for assaying blind or visually-impaired users. This may also be preferred as advantageous to garner a more full picture of the patient's audio, visual, and even tactile responsiveness to cognitive testing protocols.

As used herein, the term "Memory" denotes computer-readable memory on tangible media, able to store the test protocols, receive user responses, store a response evaluation protocol, and process said user responses according to said response evaluation protocol to generate a result (or "score"). In one version of our invention, the Memory is one single piece of electronic hardware, able to perform all of the required functions.

The Memory need not be one physical unit, however. In one preferred version, the Memory which receives the patient's responses into Memory which is physically located in an Internet-capable wireless phone, while the Memory which stores the most up-to-date version of the cognitive dysfunction testing protocol, and the software to perform the complex user response evaluation, is in Memory physically located in an Internet accessible computer server. One of the advantages of our invention is that one can make it using an extremely wide variety of physical Memory configurations, as long as one provides Memory to perform each of the required functions.

As used herein, the term "computing apparatus" includes personal computer microprocessors for both stand alone computers and those connectable to an external network or software source such as the Internet. The term also includes any electronic hardware which can execute the neurological testing routine herein described.

Thus, for example, our invention can be made using a personal handheld electronic organizer, such as the PALM PILOT III (TM), PALM PILOT V (TM) or PALM PILOT VII (TM), each commercially available from Palm Computing, Inc. Inc., Santa Clara, California, a WINDOWS CE (TM) (Microsoft Corporation, Redmond, Washington) or wireless application protocol standard or blue tooth standard appliance, a wireless telephone with adequate memory, a wireless communications device connectable to an external software source (such as the Internet), or a dedicated medical device whose sole function is to execute the cognitive testing protocols. Our invention can even be made using a television set, where the television is capable of receiving test responses from the subject, via a television remote-control device, for example. This is one of the advantages of our invention - it is extraordinarily flexible, and can be easily produced in an extremely wide variety of hardware. Our invention thus can be made in various versions which are durable, portable, inexpensive, etc..., as desired by a given kind of user.

As used herein, the term "Display" denotes apparatus to render the testing protocol perceivable by the user. In our preferred version, the display is the visual display screen on a portable personal computer (or PDA device) or on a wireless telephone. One can use other visual displays, however, including television screens or projector-based systems such as one finds for visual acuity testing at the optometrist's. Further, where one uses non-visual testing protocols, the Display will necessarily entail the ability to display the non-visual information. For example, if one uses sound auditory testing protocols, then the Display will need to include audio speakers or the like.

As used herein, the term "Response Input" denotes apparatus that the test user can use to input their responses to the test protocol into the Memory. In our preferred version, the Response Input is a keyboard or personal computer "mouse." However, one can use a stulus for a hand-held computing device, punch pads or a joystick, and so forth, or other types of electronic devices (e.g., wireless telephones, handheld computing devices, touch screen displays) and non-keyboard devices as appropriate. For example, one can use a television infrared remote-control unit, where the Display is a television. The Response Input can be anything able to communicate the user's responses to the Memory.

As used herein, the term "user response analysis software" is software capable of analyzing the user's responses to the cognitive dysfunction testing protocol, to assess whether symptoms of cognitive dysfunction likely exist or are absent in the user, based on the user's responses to the cognitive dysfunction testing protocol. The user response analysis software includes a computer readable data structure on computer readable, tangible media to store both patient responses, and the statistical analysis protocols that use the patient's responses as variable inputs. Such statistical analysis allows the most information to be obtained from these responses. Used appropriately, the statistical analysis enables the user to draw more sensitive, sophisticated conclusions from the user's responses. Statistical analysis capability had not before been combined in a single system with cognitive-function data (response) gathering capability, before the immediate invention. We disclose in detail below our preferred version of user response analysis software The term "Output" denotes a device capable of outputting the results of the user response analysis software. In our preferred embodiment, the Output includes two components: (a) a computer display screen, the same screen used as the "Display" to display the tests to the patient; and (b) a communications device to communicate the user's test results from the user response analysis software to a Memory for storage and later retrieval. Alternatively, one may use a printer, a modem (including a wireless communication device), a disk drive, or any other combination of hardware appropriate for the given version of our invention. For example, with a blind user, the Output may be an audio speaker.

The term "communication network" includes communication networks both open (such as a ground-line telephone, a radio, or a broadcast television network or the Internet) and closed (such as an intranet or a restricted access wide area network or local area network).

As mentioned above, our invention is useful specifically for methylphenidate titration. More generally, our method is useful to assess the effect on cognitive dysfunction of many other drugs, including many other potential cognitive dysfunction drugs, including drugs to treat Alzheimer's disease (rivastigmine, lazabemide, physostigmine, idebenone, propentofylline, galantamine, and metrifomate); dementia; anxiety disorders and schizophrenia.

One advantage of our invention, however, is that its application is not limited to any certain class of drug; rather, our invention can be used to inexpensively, easily and accurately assess the impact on cognitive dysfunction of any kind of pharmaceutical. Thus, for example, our invention can be used for any drug substance, to quantify the amount of cognitive impairment the drug causes in a population of test subjects in a clinical trial, so that the potential for such impairment can be properly disclosed in the drug product's labeling.

Thus, the best mode we currently know of also includes using our invention to evaluate the cognitive dysfunction impact (or side effect) of drugs intended for non-cognitive disorders. Examples include analgesics, birth control pills or antibiotics, whether or not known for inducing drowsiness and cognitive impairment.

Cognitive Dysfunction Testing Protocols

A version of our invention particularly effective for evaluating the cognitive effect of a drug entails doing a patient assessment at least once without the drug being administered to the patient, and at least once while the patient has had the drug administered. This version entails having the patient take a series of cognitive dysfunction tests while the patient has not taken the drug, and receiving the patient's test responses, and analyzing these responses, to form a "baseline" performance level for the patient, and to also - when the patient has taken the drug - have the patient take a series of cognitive dysfunction tests, and to receive the patient's test responses, and analyze these responses to form a new performance level for the patient, and to compare these new, drug influenced responses to the patient's earlier ("baseline") responses to then form a conclusion regarding whether symptoms of probable cognitive dysfunction exist and are likely caused by the drug.

In another version of our invention, the patient is not given the test in the absence of the drug; rather, the "baseline" is a composite of the test results obtained by other people. Alternatively, the patient's drug influenced results can be compared both to a standard "baseline," and to the patient's personal results in the absence of drug usage.

In the best mode we currently know of to practice our invention, one uses cognitive dysfunction testing protocols such as the following ones. These specific protocols are protected by copyright, ©2000 Head Minder, Inc. and ©2000 Xcape, Inc.

Administration of the testing protocols is preceded by displaying an ethical statement on the privacy of the test results and a legal disclaimer. The testing protocols begin only after the user's identity is verified (S170) by a test administrator, or by the user entering a code such as their social security number and a secret password (S110). Before commencing the testing protocols, the user is informed that they should not take the tests if the user has recently used alcohol or other drugs capable of affecting cognitive ability.

Administration of the testing protocols is also preceded by gathering certain general information on the user. This information can be useful or necessary to best administer the tests and interpret the test results. This general information (S130) includes the patient's Name, the e-mail and street addresses and telephone numbers for the patient, the patient's physician, the local hospital, and the patient's legal guardian (if applicable) (S130), so that any of these can be contacted quickly in an emergency (S140). In our preferred embodiment, the apparatus has a communications device such as wireless telephone capability or a modem. Similarly, we prefer to include contact information for the patient's health insurance provider (S130), so that test information and results can be directly communicated to the insurer without intervening manual transcription. The patient's date of birth and school grade are, in our preferred embodiment, entered into the software (S130) and used (S220) to determine which version of certain testing protocols (S230) to administer (we prefer to provide certain testing protocols in several different versions (S240, S340), each version suitable for a certain age group). The Test Date can be entered automatically by the computing device if it has a timer/clock function. The patient's gender, sports played, dominant hand (right, left, ambidextrous), and known prior history of type and date of prior seizures, concussions, reading problems, special education classes, native language, etc... (S130), all can be used (S220, S320) to adjust or interpret the testing protocol results (S410, S450). A chart of pupil sizes can be included, to allow the patient (or someone else) to quantify the patient's pupil size(s) (S215, S315).

The identity and dosage regime for the drug in questions can optionally be entered. This enables one to integrate with the result analysis software, a function to advise on the suggested future dosage of the drug.

If the testing protocol is supervised by someone other than the patient, we prefer to include an electronic "signature" to be entered by the test supervisor, to create a medical record authenticating who supervised the test.

We prefer that the testing protocols themselves (S260-S285, S360-S385) be arranged or ordered to put at the very beginning those tests )S270, S370) most indicative of the most severe cognitive impairment. This enables the software to rapidly triage patients (S410, S420, S430) and indicate (S492, S494, S496), for patients with cognitive impairment which is severe, that medical intervention may be required immediately, without forcing the patient to complete each and every one of the testing protocols. Similarly, we prefer to order (S250, S350) the testing protocols (S260-S285, S360-S385) so that patients with superior cognitive function can, if desired, take a longer battery of assays (S290 to S260, S390 to S360), and obtain a statistically more accurate and precise measure of cognitive function (S410, S470).

At the beginning of the testing protocols, the user is shown the keyboard layout, and shown which keys are needed for responding (S260, S360). For each testing protocol, Screen Instructions are displayed on the Display, and the user must respond appropriately before the protocol begins (S260, S360).

Each cognitive function testing protocol comprises a series of stimuli (s270, S370) shown to the user, to which the user must respond (S280-S285, S380-S385). While it is possible to make testing protocols which use words, we now prefer to use protocols which are based on images, not words. This minimizes the data bias based on less than perfect literacy, using a nonnative language for the testing protocols, and the like.

Examples of cognitive function testing protocols (S270, S370) include the following visual testing protocols :

- Tracking Part I;
- Tracking Part II
- Incidental Learning Part I;
- Incidental Learning Part II;
- Matching;
- Response Direction Part I;
- Response Direction Part II;
- Response Inhibition;
- Memory Cabinet Learning;
- Memory Cabinet Delayed Recall;
- Scanning Speed and Accuracy;
- Reaction Time;
- Cued Reaction Time;
- Visual Memory Part I;
- Number sequencing;
- Visual Memory Part II; and
- Number recall.

These protocols are examples. Our preferred embodiment uses many different specific test protocols (S270, S370). This makes it less likely that a user will memorize (S344) a specific test protocol and the percieved "correct" responses to it (S346).

We now more fully describe each these examples of testing protocols.

Tracking Part I Testing Protocol

At the beginning of this testing protocol, the Display displays the following Screen Instructions:

You are about to see a grid with 9 spaces, just like a tic-tac-toe board. A ball will appear in one of the nine spaces. A momnent later, the ball will disappear. The ball will then reappear. If the ball appears in a **different space**, then do nothing. If the ball re-appears in the **same space** as the immediately preceding time, then press the SPACE BAR. Press the SPACE BAR when you are ready to begin.

The ball is displayed in a square for 1,500 milliseconds, followed by 500 milliseconds of all blank squares. If the ball appears in the same square two times in a row, the patient should press the space bar. The patient inputs their responses into the Response Input, and thus into the Memory, for further processing by the patient response analysis software. We prefer the testing protocol to present approximately thrity stimuli over about one minute.

Tracking Part II Testing Protocol

At the beginning of this testing protocol, the Display displays the following Screen Instructions:
You are about to see the same grid as before. This time, press the SPACE BAR if the ball appears not in the space immediately preceeding, but the space before that one.
The ball is displayed in the square for 1,500 milliseconds, followed by 500 milliseconds of blank squares. If the ball appears in the same square as the time before the previous time, the user should press the space bar. We prefer to display about sixty stimuli over about two minutes. The patient inputs their responses into the Response Input, and thus into the Memory, for further processing by the patient response analysis software. This test may be modified for patients with high cognitive functioning to require a response for the third preceeding position, rather than the second or the immediately preceeding one.

Incidental Learning Part I

At the beginning of this testing protocol, the Display displays the following Screen Instructions:
Soon you will see a series of pictures appear on the screen. Whenever you see a picture of a **plant** (such as a fruit, tree or vegetable), press the space bar. If you see a picture of anything else, then do nothing. Try to be fast without making mistakes. You are being timed on how fast you respond. Press the space bar when you are ready to begin. The Display then displays pictures of plants, animals, and everyday objects. Each picture is displayed for 2 seconds, followed by 1 seconds of blank screen. The patient inputs their responses into the Response Input, and thus into the Memory, for further processing by the patient response analysis software. We prefer to dislay about fourty stimuli (about ten plants, 15 animals and 15 inanimate objects) over about two minutes.

Incidental Learning Part II

At the beginning of this testing protocol, the Display displays the following Screen Instructions:
Soon, you will see a series of pictures.- Some are from the series you saw a few minutes ago, while some are new. When you see a picture that you recognize from a few moments ago, press the space bar. If you see a picture that you have not seen before, then do nothing. Try to be fast without making mistakes. You are being timed on how fast you are. Press the space bar when you are ready to begin.
The Display then displays pictures of plants, animals and everyday objects. About twenty images from the Incidental Learning Part I are repeated. Each picture is displayed for 2.0 seconds, followed by 1.0 second of blank screen. The pat
In our preferred embodiment, separate statistics for animate and inanimate picture responses are collected and compared. We prefer about fourty stimuli over about two minutes. We prefer to give this test after Incidental Learning Part I and another, intervening task.

Matching

At the beginning of this testing protocol, the Display displays the following Screen Instructions:
You are about to see, for ten seconds, ten matching pairs of shapes laid out in a grid. Study the shapes' locations. The shapes will then be hidden under small squares. Once the shapes are hidden, use your mouse to click on any square. The shape hidden beneath the square will appear. Then, use your mouse to click on the square that you think covers the matching shape. If you do not find the matching shape, then both shapes will be covered again. Repeat the process until you find all the matching pairs. Try to make all the matches in as few tries as possible. You will not be timed. Press the space bar when you are ready to begin.

The user must find ten matching pairs of shapes. All pairs are initially displayed for ten seconds, and then covered. In the example above, the Display displays one shapes. The user must then try to find the location of the other. The patient inputs their responses into the Response Input, and thus into the Memory, for further processing by the patient response analysis software. If the user is correct, both shapes in the pair stay uncovered. Otherwise, both will be covered up again. The test continues until all matches are made or until the user attempts fourty guesses. There is no time limit.

Respionse Direction Part I

At the beginning of this testing protocol, the Display displays the following Screen Instructions:

Soon, you will see numbers appear briefly on the screen. Place your left index finger on the 1 key and your right index finger on the 0 key. When you see the number "1" displayed on your screen, press number 1 on your keyboard. When you see the number "0" displayed on your screen, press number 0 on your keyboard. If you see any other number, do nothing. Try to be fast without making mistakes. You are being timed on how fast you respond. Press the spacebar when you are ready to begin.

The Display then displays a number for about 0.5 seconds, followed by 1.5 seconds of blank screen. The patient inputs their responses into the Response Input, and thus into the Memory, for further processing by the patient response analysis software. Responses can occur anytime before the next digit is displayed. We prefer displaying about sixty stimuli over about two minutes.

Response Direction Part II

At the beginning of this testing protocol, the Display displays the following Screen Instructions:

Soon, you will see numbers appear briefly on the screen. Place your left index finger on the 1 key and your right index finger on the 0 key. Do the inverse of what you did on the last test. That is, when you see the number "1" displayed on your screen, press **number 0** on your keyboard. When you see the number "0" displayed on your screen, press **number 1** on your keyboard. If you see any other number, do nothing. Try to be fast without making mistakes. You are being timed on how fast you respond. Press the spacebar when you are ready to begin.

The Display then displays a number for about 0.5 seconds, followed by 1.5 seconds of blank screen. Responses can occur anytime before the next digit is displayed. The patient inputs their responses into the Response Input, and thus into the Memory, for further processing by the patient response analysis software. We prefer displaying about sixty stimuli over about two minutes.

Response Inhibition

At the beginning of this testing protocol, the Display displays the following Screen Instructions:

Shortly, you will see a series of pictures. Press the space bar every time you see a picture except if it is of an animal. Press the spacebar as fast as you can. You are being timed. Remeber, press the space bar every time you see a picture except if it is an animal. Press the space bar when you are ready to begin.

The Display then displays pictures of objects, plants, and animals. Each picture is displayed for 2.0 seconds, followed by 1.0 seconds of blank screen. The patient inputs their responses into the Response Input, and thus into the Memory, for further processing by the patient response analysis software. We prefer to use about sixty five stimuli over about 3.3 minutes.

Memory Cabinet Learning

At the beginning of this testing protocol, the Display displays the following Screen Instructions:

In a moment, you will see a cabinet with nine commmon objects placed on different shelves. You will have twenty seconds to memorize where each object is stored. Study hard. Doors will then close to cover the objects and you will be asked to find them, one at a time. You can do this by either (A) pressing the number key (1-9) on your keyboard that is the same as the door where you think the object is hidden, or (B) pointing and clicking your computer's mouse on the door where you think the object is hiddeen. If you make a mistake, then the test will remind you where the object is, so that you can find it later. You will be asked to find each object a total of four times. Press the space bar when you are ready to begin.

The user must memorize the locatinos of nine common objects. In one version, we prefer to use toys as objects. The locations are randomly generated for each user, to minimize users being able to "memorize" the locations. The user is queried about the locations one at a time. The patient inputs their responses into the Response Input, and thus into the Memory, for further processing by the patient response analysis software. After one round (9 queries), there is a second 10 second display, and then the process repeats. This continues until the user has been asked for a locatin of each object four times. If the user guesses incorrectly, then the correct locatin is briefly shown. If he guesses correctly, the Display displays "correct." Statistics are collected for each round. There is no time limit.

Memory Cabinet Delayed Recall

At the beginning of this testing protocol, the Display displays the following Screen Instructions:
A few moments ago, you saw a cabinet with nine objects placed on different shelves. In a moment, you will be asked to find those items one at a time, just like you did before. This time, you will not see the objects first, and you will not be told if you are right or wrong. Press the spacebar when you are ready to begin.
There is no initial display of objects. The user must recall their locations from Memory Cabinet Learning. The patient inputs their responses into the Response Input, and thus into the Memory, for further processing by the patient response analysis software. There is only one round of queries, and no feedback about the correctness of a response. This test must be given after Memory Cabinet Learning, and preferrably after another intervening task.

Scanning Speed and Accuracy

At the beginning of this testing protocol, the Display displays the following Screen Instructions:
Now, look at the sample shapes below. The shapes are in two groups. If both the shapes on the left hand side of the line are also on the right hand side of the line, press the space bar ONCE. If the shapes are not BOTH on the right hand side, then press the space bar TWICE. You only get one chance for each item. Remember - press ONCE for yes and TWICE for no. Work as fast as you can without making any mistakes. Press the space bar when you are ready to begin.
The Display then displays to the patient two groupings of symbols, one on the left side of the Display and one grouping on the right side of the Display, like this:

$$¿ \ \tilde{A} \qquad \qquad § \ ¶ \ \check{s} \ ? \ § \ ¿ \ \tilde{A} \ \mathring{A}$$

Each of approximately thirty groupings of symbols appears separately. The patient inputs their responses into the Response Input, and thus into the Memory, for further processing by the patient response analysis software. We prefer to fix the time required at ninety seconds.
An alternate version of this testing protocol is to ask the patient to hit the number "1" key if one target shape is present on the right side of the Display. and the number "2" key if both target shapes are present there.

Reaction Time

At the beginning of this testing protocol, the Display displays the following Screen Instructions:
Look at the sample white circle below. Each time that you see the white circle, press the space bar. Try and be quick without making mistakes. Press the space bar when you are ready.
The Display then displays a series of pictures to the patient, using a ratio of 1 "target" image (in this example, a white circle) for every several non-target images (in this example, nonwhite circles) displayed.
The patient inputs their responses into the Response Input, and thus into the Memory, for further processing by the patient response analysis software. In our preferred version, in the Reaction Time testing protocol, the visual stimulus duration is 1.5 seconds, followed by 0.5 seconds of blank screen. The patient's response can therefore occur any time within the 1.5 second stimulus, but is not allowed thereafter.
For the personal computer versions of our inventions (in contrast to, for example, the PALM PILOT (TM) based versions), we prefer using certain software operating systems most able to accommodate the rapid response time limits of this testing protocol. Personal computer timers operate independently of the microprocessor speed. Thus, using a 266 MHz microprocessor, or a 450 MHz one, does not affect timer speed. However, different operating systems have different rates of updating the timer. Thus, on WINDOWS 3.11 (TM), WINDOWS 95 (TM) and WINDOWS 98 (TM) (each commercially available from Microsoft Corp., Redmond, Washington), the timer is updated only 18.2 times per second, resulting in a maximum resolution of +27 milliseconds. For many testing protocols, this will have no significant impact.

Cued Reaction Time

At the beginning of this testing protocol, the Display displays the following Screen Instructions:
Press the space bar only when the white circle is displayed after a black square is displayed. Do not press the space bar if the white circle is displayed after any other shape, nor any other color of square. Remember, press the space bar only when the white circles is displayed after a black square. Try and be quick without making mistakes. Press the space bar when you are ready to start.
The Display then displays the black square followed by white circle pair, in a ratio of 1:6 with total other stimuli. The ratio of the target (white circle) with target primer (black square), to target without a target primer, is 2:1. The patient inputs their responses into the Response Input, and thus into the Memory, for further processing by the patient response analysis software. This portion of the testing protocol takes 3 minutes.

Visual Memory Part I

At the beginning of this testing protocol, the Display displays the following Screen Instructions:
Now, you will see a series of pictures appear on the display. Sometimes, you will see a picture a second time. Each time you see a picture for the second time, press the space bar. Press the space bar when you are ready to begin.
The Display then displays a series of pictures, as for example:

Each of the single forty pictures is displayed for two seconds. Of the forty pictures, twenty are repeated and twenty are not, for a test time of two minutes. The patient inputs their responses into the Response Input, and thus into the Memory, for further processing by the patient response analysis software.

Number Sequencing

At the beginning of this testing protocol, the Display displays the following Screen Instructions:
Below is a key that pairs the numbers 1 through 9 with svmbols. Beneath the kev, vou will see a series of symbols with empty boxes underneath. Fill in the correct numbers for each symbol using the numeric keypad. If you make a mistake, just keep going. Try and fill in as many numbers as you can. Press the space bar once to begin.
The Display then displays, for ninety seconds, a screen like this:

**KEY**

| ß | ø | Þ | ‡ | ‰ | « |
|---|---|---|---|---|---|
| 1 | 2 | 3 | 4 | 5 | 6 |

**TEST**

| ø | ‰ | ø | « | Þ | ‡ |
|---|---|---|---|---|---|
| | | | | | |
| Þ | ø | ‰ | ‡ | ‰ | ø |
| | | | | | |

The patient inputs their responses into the Response Input, and thus into the Memory, for further processing by the patient response analysis software. In our preferred embodiment, we use animal silhouettes rather than typographic symbols, but words, numbers, and any other visual indicia are all acceptable.

Visual Memory Part II

At the beginning of this testing protocol, the Display displays the following Screen Instructions:

Just a few moments ago, you saw a list of pictures. Some you saw once, others twice. Press the space bar when you see a picture that you recognize from before. It can be one that you just saw once, or one that you saw twice. Press the space bar when you are ready to begin.

The Display then displays a series of pictures, one every two seconds. All the forty pictures from the Visual Memory Part II testing protocol are displayed, in addition to twenty new pictures, over a two minute total time. The patient inputs their responses into the Response Input, and thus into the Memory, for further processing by the patient response analysis software.

Number Recall

At the beginning of this testing protocol, the Display displays the following Screen Instructions:

Now, you will see a series of numbers appear on the display, followed by a display screen with some blanks on it. Using the number keys, enter the numbers in the blanks in exactly the same order as you see them. You can use the backspace key to change your answer if you think you have made a mistake. Press the space bar when you are ready to begin.

The Display then displays a series of individual numerals, one numeral at a time, like this:

**5     3**

Each group of numerals is displayed for 750 milliseconds. The first groups displayed consist of only two numerals. Latter groups consist of longer and longer groups of numerals:

**7     4     8     2     9** The patient inputs their responses into the Response Input, and thus into the Memory, for further processing by the patient response analysis software. The testing protocol continues until the patient makes two consecutive errors on the same level of difficulty (i.e., two consecutive errors with numeral groups having the same quantity of numerals in them). When the patient makes these two consecutive errors, the testing protocol stops. Number sequencing

At the beginning of this testing protocol, the Display displays the following Screen Instructions:

Now, you will see a group of numerals appear on the display, followed by a display screen with some blanks on it. Using the numeric keypad, enter the numbers in the blanks in ASCENDING order. That is, order them from lowest to highest. You can use the backspace key to change your answer if you think you have made a mistake. Press the space bar when you are ready to begin.

The Display then displays a group of numbers, like this:

**5     3     4**

The patient inputs their response (the correct response would be "3 4 5" in the immediate example) into the Memory. Each group of numbers is presented for two seconds. The first groups displayed consist of only three numerals. Latter groups consist of longer and longer groups of numerals:

**2     8     3     1     9**

The patient inputs their responses into the Response Input, and thus into the Memory, for further processing by the patient response analysis software. The testing protocol is discontinued when the patient makes two consecutive errors on the same level of difficulty (i.e., with two consecutive number groups having the same quantity of numbers in them). This test portion takes approximately three minutes.

Summary

On completion of the testing protocol(s) (S290, S390), the patient is informed that the testing is complete. Either after completion of all protocols, or during the test process, the patient's response data (S285, S385) is used as variable inputs (S410, S450) in the patient response analysis software.

Patient Response Analysis Software

The user's results (S410, S450) for the cognitive dysfunction testing protocols are then analyzed statistically (S420, S460), to obtain the most information from them. In our invention, the statistical analysis capability is integrated into the system. This is done by incorporating directly into our system, patient response analysis software (S410-S470). The patient response analysis software uses as variable inputs the testing protocol results discussed above. The patient response analysis software then statistically analyzes these responses and calculates certain values for each specific testing protocol, the values for certain protocols combined, and the values for all the protocols combined. We discuss each in turn.

In our preferred version, we use the following statistical analysis protocols. This compilation of these statistical protocols is protected by copyright, ©1999 Xcape, Inc.

Individual Testing Protocols

For each separate cognitive dysfunction testing protocol, the patient response analysis software calculates:

t = Average Response Time
y = correct responses
O = Errors of Omission (misses or errors)
C = Errors of Commission (false positives, if applicable)

From these, the following ratios can be derived:

$$\text{Proficiency Index} = (O + C) / t$$

$$\text{Discriminability} = 100 \times [1 - (O + C / \text{Number of Stimuli})]$$

Note that in our preferred embodiment, neither Proficiency Index nor Discriminability are used.

Response Bias = (C - O) / (C + O); if no errors then = 0.
Response Variability = mean standard deviation of response times.

Response Variability is calculated for the continuous performance test protocol (s) (e.g., the "Go No-Go Test" above) only.

$$\text{Q Level} = y - C - O$$

$$\text{Retention Index} = 100 \times \text{Delayed Recall} / \text{Immediate Recall}.$$

Retention Index is calculated for the memory tests only.

Test - Retest Correlation

If baseline data is available (either from a patient pool, or specifically from a prior test administered to that patient), then the patient response analysis software can also calculate, for each of the above values, the correlation between a given baseline test value ("a") and the value obtained in a subsequent test ("b"). We denote this correlation here as "r(ab)."

$$r(ab) = S(ab) / \text{sqrt} [S(aa) \times S(bb)]$$

S = Sigma = standard deviation
Mu = mean
S(aa) = SUM [(a - mean (a)) ^ 2]
S(bb) = SUM [(b - mean (b)) ^ 2]
S(ab) = SUM [abs[(a - mean(a)) x (b - mean (b))]]

Selectively Combined Testing Protocol Analysis

In addition to analyzing data for each testing protocol separately (S430), the patient response analysis software combines the results of certain testing protocols (S450) for certain analyses.

General Attention = total correct responses for Number sequencing and Number recall protocols.

Attention Consistency = the weighted number of digits in Number sequencing and Number recall.

Attention Accuracy = (Discriminability for Response Speed + Discriminability for Response Cueing and Inhibition) / 2.

Attention Efficiency = (Proficiency for Response Speed + Proficiency for Response Cueing and Inhibition) / 2.

Processing Speed Accuracy = (Q Level for Symbol Scanning + Q Level for Number sequencing) / 2.

Processing Speed Efficiency = (Proficiency for Symbol Scanning + Proficiency for Number sequencing) / 2.

Memory Accuracy = (y for Visual Memory Part I + y for Visual Memory Part II) / 2.

Memory Efficiency = (Proficiency for Visual Memory Part I + Proficiency for Visual Memory Part II) / 2.

Reaction Time Index = average reaction time for Response Speed + average reaction time for Response Cueing and Inhibition.

Processing Speed Index = average reaction time for Symbol Scanning + average reaction time for Number sequencing.

Complex Reaction Time = average reaction time for Visual Memory Part II + average reaction time for Response Cueing and Inhibition.

Total Combined Testing Protocol Values

For all cognitive dysfunction testing protocols combined, the patient response analysis software calculates:

Overall Speed
Overall Accuracy
Overall Proficiency = Overall Speed / Overall Accuracy

In our preferred embodiment, the speed, accuracy and efficiency result indices are generated at the domain level; that is to say, if one cognitive dysfunction testing protocol at baseline is outside the normal range, the software can still generate a statistically meaningful score. If this is not done, then if a patient does not understand the instructions, or has attention deficit disorder, or is disturbed by a telephone call during the test, then that patient's erroneous results will create systematic error which can distort the general score.

Reliable Change Index

The patient response analysis software then calculates a "reliable change index" (S430, S470). The reliable change index describes the change from the baseline value, which change is statistically reliable. There are many ways known in the art of statistics to calculate reliable changes (S420, S460). We prefer to calculate the reliable change index (or "RCI") as follows:

$$RCI = X(b) - X(a) / s(d)$$

X(a) = the baseline value
X(b) = the immediate value
s(d) = the standard difference for the sub test calculation, as calculated above.
p = the probability of error

Regardless of the specific statistical method used to calculate the RCI, the RCI threshold values should, optimally, be set considering generally accepted statistical principles. One tailed and other tests are possible. In our preferred version, the positive and negative RCI threshold values are derived from accepted medical neurology standards. Examples are given in Hinton-Bayre, A.D., *"Concussion in Contact Sports: Reliable Change Indices of Impairment and Recovery*," Journal of Clinical and Experimental Neuropsychology, v.21, pp.70-86 (1999). Other values may, however, be used.

For a one tailed test, we prefer to use a negative RCI threshold value of -1.65, with p < 0.05. We similarly prefer to use a positive RCI threshold value of-1.04 with p < 0.15. Using these amounts, a test result with an RCI < -1.65, indicates symptoms of cognitive dysfunction likely exist in the patient (and the pharmaceutical dosage may need to be adjusted). By contrast, an RCI ≥ -1.04 indicates symptoms of cognitive dysfunction likely do not exist in the patient (and the pharmaceutical dosage may not need to be adjusted). Other threshold values may, of course, be used.

If an RCI value falls outside its negative RCI threshold range, or if there is at least one active cognitive dysfunction symptom in the pre-testing protocol user survey, then the user response analysis software indicates that symptoms of cognitive dysfunction *likely exist* in the user. Conversely, if all RCI values are within the positive RCI threshold ranges and if there is no active trauma symptom, then the user response analysis software indicates that symptoms of cognitive dysfunction *likely do not* exist in the user. If at least one RCI value falls inside the negative RCI threshold range but outside the positive RCI threshold range, and if there is no active trauma symptom, then the user response analysis software indicates that symptoms of cognitive dysfunction *may exist* in the user.

For certain applications (clinical trials, for example), patients can establish a "baseline" score (S210-S295) before the drug trial begins, or before drug use occurs, and use this baseline to compare to later (S310-S395) scores. In such a use, RCI scores which fall too far outside the normal range (we prefer less than two standard deviations from the mean) are rejected, as drug induced cognitive impairment, even severe, may not statistically lower a score which is already quite low. Thus, we prefer to not have such users (nor their physicians) rely on these scores to allow a user to continue to take a potentially harmful dose of a drug. Low baseline scores could be due to a number of factors including a history of learning problems, distraction and confusion over the instructions, and a conscious attempt to fake a lowered score, in order to manipulate future results.

SUMMARY

Although the present invention has been described in considerable detail with reference to certain preferred versions, other versions are possible. Therefore, the spirit and scope of the appended claims should not be limited to only the description of our preferred versions contained in the foregoing discussion. The features disclosed in this specification, and the accompanying claims and abstract, may be replaced by alternative features serving the same equivalent or similar purpose, unless expressly stated otherwise. Thus, unless expressly stated otherwise, each feature disclosed is one example only of a generic series of equivalent or similar features.

As used in the claims appended, the word "a" includes the singular as well as the plural. The phrase "in communication with" entails both direct communication and indirect communication via one or more intermediary pieces (e.g., microprocessors, cables, etc...). The terms "clinical study," "drug substance," "drug product," "label," "labeled," "labeling," and "New Drug Application" are used herein as defined in the Federal Food, Drug & Cosmetic Act and the regulations in effect from time to time thereunder. Claims reciting a drug "labeled for use with cognitive dysfunction testing protocol," which claims recite limitations on the cognitive dysfunction testing protocol, require that the drug label refer to the cognitive dysfunction testing protocol,. but do not require that the label recite each of the claim limitations on the cognitive dysfunction testing protocol. As used in the claims appended, the term "treat" includes both minimizing the unwanted symptoms of the cited ailment, and minimizing the existence or causes of that ailment.

**Claims**

1. A drug product labeled for use with computer readable, tangible Memory, said Memory comprising:

   a. Testing Memory on computer readable, tangible media, said Testing Memory storing cognitive dysfunction testing protocols,
   b. Response Memory on computer readable, tangible media, said Response Memory able to receive and store user responses to said neurological pathology testing protocols, and
   c. Statistical Memory on computer readable, tangible media, said Statistical Memory storing user response analysis software.

2. The drug product of claim 1:

   said Response Memory comprising a "baseline" which is not the results of said user,
   whereby said user response analysis software can compare (i) the cognitive function testing protocol results for said user to (ii) said baseline result.

3. The drug product of claim 1:

   said Response Memory comprising said user's cognitive pathology testing protocol results taken on at least two different times, and
   wherein said user response analysis software can compare said results.

4. The drug product of claim 3:

   wherein said data structure comprises responses for said user taken on at least two different times,
   whereby said user response analysis software can thereby compare for said user, responses of said user at a plurality of times.

**5.** The drug product of claim 4:
   wherein said plurality of times include at least one time in the absence of said drug substance, and at least one time in the presence of said drug substance.

**6.** The drug product of claim 5:
   wherein said user response analysis software calculates a reliable change index.

**7.** The drug product of claim 6:
   wherein said user response analysis software further indicates if the dosage of said drug substance for said patient should be modified.

**8.** The drug product of claim 5:

   wherein said Memory is communicably connected to a communications device,
   whereby said user's cognitive dysfunction testing protocol responses may be communicated over said communication network.

**9.** The drug product of claim 8:
   wherein said communication network consists essentially of the Internet.

**10.** The drug product of claim 8:
   wherein said communication network consists essentially of a wireless telephone network.

**11.** The drug product of claim 5:
   wherein said drug product is labeled for use with a focal brain disorder of a higher function.

**12.** The drug product of claim 9:
   wherein said drug product is labeled for use with a global-diffuse cerebrum disorder.

**13.** The drug product of claim 12:
   wherein said global-diffuse cerebrum disorder is selected from the group consisting of: disorder caused by mechanical trauma, subarachnoid hemorrhage, a supratentorial mass lesion, a subtentorial lesion, multi-infarct dementia, chronic communicating hydrocephalus with "normal" pressure, and dementia associated with severe head injury.

**14.** The drug product of claim 12:
   wherein said global-diffuse cerebrum disorder is selected from the group consisting of: anoxia, ischemia, epilepsy, postictal states and psychomotor status epilepticus.

**15.** The drug product of claim 12:
   wherein said global diffuse cerebrum disorder is caused by central nervous system infection or toxin (exogenous or endogenous).

**16.** The drug product of claim 12:
   wherein said global diffuse cerebrum disorder is a dementia.

**17.** The drug product of claim 16:
   wherein said dementia comprises chronic drug - alcohol nutritional abuse.

**18.** The drug product of claim 16:
   wherein said dementia is selected from the group conisiting of Huntington's disease, dementia paralytica, Creutzfeldt - Jacob disease, Wilson's disease, parkinsonism.

**19.** The drug product of claim 16:
   wherein said dementia is selected from the group consisting of: multiple sclerosis, amytropic lateral sclerosis and a demyelinating disease.

**20.** The drug product of claim 16:
wherein said dementia is selected from the group consisting of: Alzheimer's presenile dementia, Alzheimer's senile onset dementia, Pick's disease and simple (idiopathic) presenile dementia.

**21.** The drug product of claim 6:
wherein said drug product is labeled to treat a seizure disorder.

**22.** The drug product of claim 6:
wherein said drug product is labeled to treat cerebrovascular disease.

**23.** The drug product of claim 6:
wherein said drug product is labeled to treat a psychological disorder.

**24.** The drug product of claim 23:
wherin said psychological disorder is selected from the group consisting of: schizophrenia, depression, hyperactivity, phobias, panic attacks, anxiety, overeating, personality disorders, obsessive-compulsive disorders, hysteria and paranoia.

**25.** The drug product of claim 6:
wherein said drug product is labeled to treat attention deficit disorder.

**26.** The drug product of claim 6:
wherein said drug product is methylphenidate.

**27.** The drug product of claim 6:
wherein said drug product is adderall.

**28.** The drug product of claim 6:
wherein said drug product is selected from the group consisting of: rivastigmine, lazabemide, physostigmine, idebenone, propentofylline, galantamine and metrifomate.

**29.** The drug product of claim 6:
wherein said drug product is authorized to be marketed pursuant to an approved New Drug Application.

**30.** A system comprising:

A. a drug product, and
B. computer readable, tangible Memory, said Memory comprising:

a. Testing Memory on computer readable, tangible media, said Testing Memory storing cognitive dysfunction testing protocols,
b. Response Memory on computer readable, tangible media, said Response Memory able to receive and store user responses to said neurological pathology testing protocols, and
c. Statistical Memory on computer readable, tangible media, said Statistical Memory storing user response analysis software.

**31.** The system of claim 30:

said Response Memory comprising a "baseline" which is not the results of said user,
whereby said user response analysis software can compare (i) the cognitive function testing protocol results for said user to (ii) said baseline result.

**32.** The system of claim 30:

said Response Memory comprising said user's cognitive pathology testing protocol results taken on at least two different times, and
wherein said user response analysis software can compare said results.

**33.** The system of claim 32:

wherein said data structure comprises responses for said user taken on at least two different times, whereby said user response analysis software can thereby compare for said user, responses of said user at a plurality of times.

**34.** The system of claim 33:
wherein said plurality of times include at least one time in the absence of said drug substance, and at least one time in the presence of said drug substance.

**35.** The system of claim 34:
wherein said user response analysis software calculates a reliable change index.

**36.** The system of claim 35:
wherein said user response analysis software further indicates if the dosage of said drug substance for said patient should be modified.

**37.** The system of claim 34:

wherein said Memory is communicably connected to a communications device, whereby said user's cognitive dysfunction testing protocol responses may be communicated over said communication network.

**38.** The system of claim 37:
wherein said communication network consists essentially of the Internet.

**39.** The system of claim 37:
wherein said communication network consists essentially of a wireless telephone network.

**40.** The system of claim 33:
wherein said drug product is labeled for use with a focal brain disorder of a higher function.

**41.** The system of claim 38:
wherein said drug product is labeled for use with a global-diffuse cerebrum disorder.

**42.** The system of claim 41:
wherein said global-diffuse cerebrum disorder is selected from the group consisting of: disorder caused by mechanical trauma, subarachnoid hemorrhage, a supratentorial mass lesion, a subtentorial lesion, multi-infarct dementia, chronic communicating hydrocephalus with "normal" pressure, and dementia associated with severe head injury.

**43.** The system of claim 41:
wherein said global-diffuse cerebrum disorder is selected from the group consisting of: anoxia, ischemia, epilepsy, postictal states and psychomotor status epilepticus.

**44.** The system of claim 41:
wherein said global diffuse cerebrum disorder is caused by central nervous system infection or toxin (exogenous or endogenous).

**45.** The system of claim 41:
wherein said global diffuse cerebrum disorder is a dementia.

**46.** The system of claim 45:
wherein said dementia comprises chronic drug - alcohol nutritional abuse.

**47.** The system of claim 45:
wherein said dementia is selected from the group conisiting of Huntington's disease, dementia paralytica, Creutzfeldt - Jacob disease, Wilson's disease, parkinsonism.

**48.** The system of claim 45:

    wherein said dementia is selected from the group consisting of: multiple sclerosis, amytropic lateral sclerosis and a demyelinating disease.

**49.** The system of claim 45:

    wherein said dementia is selected from the group consisting of: Alzheimer's presenile dementia, Alzheimer's senile onset dementia, Pick's disease and simple (idiopathic) presenile dementia.

**50.** The system of claim 35:

    wherein said drug product is labeled to treat a seizure disorder.

**51.** The system of claim 35:

    wherein said drug product is labeled to treat cerebrovascular disease.

**52.** The system of claim 35:

    wherein said drug product is labeled to treat a psychological disorder.

**53.** The system of claim 52:

    wherin said psychological disorder is selected from the group consisting of: schizophrenia, depression, hyperactivity, phobias, panic attacks, anxiety, overeating, personality disorders, obsessive-compulsive disorders, hysteria and paranoia.

**54.** The system of claim 35:

    wherein said drug product is labeled to treat attention deficit disorder.

**55.** The system of claim 35:

wherein said drug product is methylphenidate.

**56.** The system of claim 35:

wherein said drug product is adderall.

**57.** The system of claim 35:

    wherein said drug product is selected from the group consisting of: rivastigmine, lazabemide, physostigmine, idebenone, propentofylline, galantamine and metrifomate.

**58.** The system of claim 35:

    wherein said drug product is authorized to be marketed pursuant to an approved New Drug Application.

**59.** A process for monitoring a pharmaceutical's effect on cognitive dysfunction, said method comprising the steps of:

    A. administering to a patient a drug substance, and
    B. providing to said patient:

        a. Testing Memory on computer readable, tangible media, said Testing Memory storing cognitive dysfunction testing protocols,
        b. Response Memory on computer readable, tangible media, said Response Memory able to receive and store user responses to said neurological pathology testing protocols, and
        c. Statistical Memory on computer readable, tangible media, said Statistical Memory storing user response analysis software.

**60.** The process of claim 59:

    said Response Memory comprising a "baseline" which is not the results of said user,
    whereby said user response analysis software can compare (i) the cognitive function testing protocol results for said user to (ii) said baseline result.

**61.** The process of claim 59:

said Response Memory comprising said user's cognitive pathology testing protocol results taken on at least two different times, and

wherein said user response analysis software can compare said results.

**62.** The process of claim 61:

wherein said data structure comprises responses for said user taken on at least two different times, whereby said user response analysis software can thereby compare for said user, responses of said user at a plurality of times.

**63.** The process of claim 62:

wherein said plurality of times include at least one time in the absence of said drug substance, and at least one time in the presence of said drug substance.

**64.** The process of claim 63:

wherein said user response analysis software calculates a reliable change index.

**65.** The process of claim 64:

wherein said user response analysis software further indicates if the dosage of said drug substance for said patient should be modified.

**66.** The process of claim 63:

wherein said Memory is communicably connected to a communications device, whereby said user's cognitive dysfunction testing protocol responses may be communicated over said communication network.

**67.** The process of claim 66:

wherein said communication network consists essentially of the Internet.

**68.** The process of claim 66:

wherein said communication network consists essentially of a wireless telephone network.

**69.** The process of claim 63:

wherein said drug product is labeled for use with a focal brain disorder of a higher function.

**70.** The process of claim 67:

wherein said drug product is labeled for use with a global-diffuse cerebrum disorder.

**71.** The process of claim 70:

wherein said global-diffuse cerebrum disorder is selected from the group consisting of: disorder caused by mechanical trauma, subarachnoid hemorrhage, a supratentorial mass lesion, a subtentorial lesion, multi-infarct dementia, chronic communicating hydrocephalus with "normal" pressure, and dementia associated with severe head injury.

**72.** The process of claim 70:

wherein said global-diffuse cerebrum disorder is selected from the group consisting of: anoxia, ischemia, epilepsy, postictal states and psychomotor status epilepticus.

**73.** The process of claim 70:

wherein said global diffuse cerebrum disorder is caused by central nervous system infection or toxin (exogenous or endogenous).

**74.** The process of claim 70:

wherein said global diffuse cerebrum disorder is a dementia.

**75.** The process of claim 74:

wherein said dementia comprises chronic drug - alcohol nutritional abuse.

**76.** The process of claim 74:

wherein said dementia is selected from the group conisiting of Huntington's disease, dementia paralytica, Creutzfeldt - Jacob disease, Wilson's disease, parkinsonism.

**77.** The process of claim 74:

wherein said dementia is selected from the group consisting of: multiple sclerosis, amytropic lateral sclerosis and a demyelinating disease.

**78.** The process of claim 74:

wherein said dementia is selected from the group consisting of: Alzheimer's presenile dementia, Alzheimer's senile onset dementia, Pick's disease and simple (idiopathic) presenile dementia.

**79.** The process of claim 64:

wherein said drug product is labeled to treat a seizure disorder.

**80.** The process of claim 64:

wherein said drug product is labeled to treat cerebrovascular disease.

**81.** The process of claim 64:

wherein said drug product is labeled to treat a psychological disorder.

**82.** The process of claim 81:

wherin said psychological disorder is selected from the group consisting of: schizophrenia, depression, hyperactivity, phobias, panic attacks, anxiety, overeating, personality disorders, obsessive-compulsive disorders, hysteria and paranoia.

**83.** The process of claim 64:

wherein said drug product is labeled to treat attention deficit disorder.

**84.** The process of claim 64:

wherein said drug product is methylphenidate.

**85.** The process of claim 64:

wherein said drug product is adderall.

**86.** The process of claim 64:

wherein said drug product is selected from the group consisting of: rivastigmine, lazabemide, physostigmine, idebenone, propentofylline, galantamine and metrifomate.

**87.** The process of claim 64:

wherein said drug product is authorized to be marketed pursuant to an approved New Drug Application.

**88.** A product-by-process article of manufacture comprising a clinical study made by using the process of any of claims 59 to 87.

**89.** A computer program comprising program instructions for causing a computer to perform step B of the process of any of claims 59 to 87.

**90.** A computer program as claimed in claim 89, embodied on a record medium.

**91.** A computer program as claimed in claim 89 stored in a computer memory.

**92.** A computer program as claimed in claim 89, embodied in a read-only memory.

**93.** A computer program as claimed in claim 89, carried on an electrical carrier signal.

**94.** A drug product as claimed in any of claims 1 to 29 or a system as claimed in any of claims 30 to 58, wherein the user response analysis software comprises a computer program as claimed in any of claims 89 to 93.

*Figure 1*

```
┌─────────────────┐
│    REGISTER     │
│      S100       │
└─────────────────┘
         │
         ▼
┌─────────────────┐
│   ADMINISTER    │
│     TEST 1      │
│      S200       │
└─────────────────┘
         │
         ▼
┌─────────────────┐
│   ADMINISTER    │
│     TEST 2      │
│      S300       │
└─────────────────┘
         │
         ▼
┌─────────────────┐
│   CALCULATE     │
│    RELIABLE     │
│     CHANGE      │
│     INDEX       │
│      S400       │
└─────────────────┘
```

*Figure 2*

SOLICIT PASSWORD S110

PASSWORD EXISTS? S120 — NO

YES

COMPARE TO PASSWORD DATA STRUCTURE S160

VALID PASSWORD? S170 — NO → SOLICIT DATA FOR REGISTRATION S130

YES

Patient Contacts
  Name
  Address
  Telephone
  e-mail
Emergecy Contacts
  Physician Name
  Physician Telephone
  Parent Name
  Parent Telephone
Patient Records Contacts
  Social Security Number
  Insurance Plan Number

ALLOW TESTING S180 ← ASSIGN PASSWORD S150 ← STORE REGISTRATION DATA S140

## *Figure 3*

SOLICIT
THIRD PARTY
OBSERVATIONS
S215

Physical Assessment
Pupil Diameter
Time Unconscious
Consciousness Assessment
Count Backwards
Last Thing Remembered

PASSWORD
ENTRY
S210

RETRIEVE
PATIENT
DATA
S220

STORE
OBSERVATIONS
S225

SELECT TYPE(S)
OF TESTS TO
ADMINISTER
S230

SELECT
TEST(S)
S250

SEARCH TEST
LIBRARY
S240

DISPLAY TEST
INSTRUCTIONS
S260

DISPLAY
TEST
S270

STORE
RESPONSES
S285

RECEIVE
RESPONSES
S280

OUTPUT
RESULTS
S295

ADDITIONAL
TESTING
DESIRED?
S290

NO

YES

## Figure 4

SOLICIT
THIRD PARTY
OBSERVATIONS
S315

Physical Assessment
Pupil Diameter
Time Unconscious
Consciousness Assessment
Count Backwards
Last Thing Remembered

PASSWORD
ENTRY
S310

RETRIEVE
PATIENT
DATA
S320

STORE
OBSERVATIONS
S325

SELECT TYPE(S)
OF TESTS TO
ADMINISTER
S330

SELECT
TEST(S)
S350

SEARCH TEST
LIBRARY
S340

DISPLAY TEST
INSTRUCTIONS
S360

NONE

PATIENT
EXPERIENCE
WITH TEST?
S342

DISPLAY
TEST
S370

EXPERIENCED

STORE
RESPONSES
S385

RECEIVE
RESPONSES
S380

NONE

PRIOR
EXPOSURE
EFFECT
S344

OUTPUT
RESULTS
S395

ADDITIONAL
TESTING
DESIRED?
S390

CREATES
BIAS

AVOID
PRIOR
TEST(S)
S346

NO

YES

*Figure 5*

```
┌──────────────┐                                              ┌──────────────┐
│  RETRIEVE    │                                              │  RETRIEVE    │
│  PATIENT     │                                              │  PATIENT     │
│  DATA FOR    │                                              │  DATA FOR    │
│  TESTS x, x+n│                                              │  TESTS x, x+n│
│  S410        │                                              │  S450        │
└──────────────┘                                              └──────────────┘
        │                                                             │
        ▼                                                             ▼
┌──────────────┐                                              ┌──────────────┐
│ SELECT       │                                              │ SELECT       │
│ RELIABLE     │                                              │ RELIABLE     │
│ CHANGE INDEX │                                              │ CHANGE INDEX │
│ CALCULATION  │                                              │ CALCULATION  │
│ METHOD       │                                              │ METHOD       │
│ S420         │                                              │ S460         │
└──────────────┘                                              └──────────────┘
        │                          YES                                │
        ▼                           ▲                                 ▼
┌──────────────┐             ◇◇◇◇◇◇◇◇◇◇◇◇             ┌──────────────┐
│ CALCULATE    │────▶        R.C.I.                    │ CALCULATE    │
│ R.C.I.       │        CALCULATED                     │ R.C.I.       │
│ S430         │        FOR EACH TEST                  │ S470         │
└──────────────┘        PROTOCOL?                      └──────────────┘
                            S440
                             │
                             NO
                             ▼
                    ┌──────────────┐
                    │ OUTPUT       │
                    │ RESULTS      │
                    │ S490         │
                    └──────────────┘
```

R.C.I. SCORES

PATIENT DIAGNOSIS DATA S494

PHARMACEUTICAL TITRATION RESULT S496